(19) Europäisches Patentamt
European Patent Office
Office européen des brevets

(11) **EP 3 317 027 B1**

(12) **FASCICULE DE BREVET EUROPEEN**

(45) Date de publication et mention de la délivrance du brevet:
**29.09.2021 Bulletin 2021/39**

(51) Int Cl.:
*B06B 3/04* *(2006.01)*    *G10K 11/30* *(2006.01)*
*A61B 8/00* *(2006.01)*    *A61B 8/08* *(2006.01)*

(21) Numéro de dépôt: **16744443.9**

(22) Date de dépôt: **30.06.2016**

(86) Numéro de dépôt international:
**PCT/FR2016/051625**

(87) Numéro de publication internationale:
**WO 2017/001781 (05.01.2017 Gazette 2017/01)**

(54) **PROCÉDÉ D'INSONIFICATION POUR OBTENIR UN CHAMP PRÉDÉTERMINÉ D'ONDES ULTRASONORES, ET PROCÉDÉ DE FABRICATION POUR RÉALISER UNE LENTILLE ULTRASONORE À CES FINS**

BESCHALLUNGSVERFAHREN ZUR ERZEUGUNG EINES VORBESTIMMTEN FELDES AUS ULTRASCHALLWELLEN UND HERSTELLUNGSVERFAHREN ZUR ERZEUGUNG EINER ULTRASCHALLLINSE FÜR SOLCH EINEN ZWECK

INSONIFICATION METHOD FOR OBTAINING A PREDETERMINED FIELD OF ULTRASONIC WAVES, AND MANUFACTURING METHOD FOR OBTAINING AN ULTRASONIC LENS FOR SUCH PURPOSE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.07.2015 FR 1556217**

(43) Date de publication de la demande:
**09.05.2018 Bulletin 2018/19**

(73) Titulaires:
• **Centre National de la Recherche Scientifique (CNRS)**
**75016 Paris (FR)**
• **INSERM (Institut National de la Santé et de la Recherche Médicale)**
**75013 Paris (FR)**

(72) Inventeurs:
• **TANTER, Mickaël**
**92220 Bagneux (FR)**
• **AUBRY, Jean-François**
**92340 Bourg la Reine (FR)**
• **DEFFIEUX, Thomas**
**95000 Cergy (FR)**
• **PERNOT, Mathieu**
**75004 Paris (FR)**

(74) Mandataire: **Osha Liang**
**2, rue de la Paix**
**75002 Paris (FR)**

(56) Documents cités:
**WO-A1-2014/176483    FR-A1- 2 843 874
US-A- 5 640 961**

• **KIM YOHAN ET AL: "Rapid prototyping fabrication of focused ultrasound transducers", IEEE TRANSACTIONS ON ULTRASONICS, FERROELECTRICS AND FREQUENCY CONTROL, IEEE, US, vol. 61, no. 9, 1 septembre 2014 (2014-09-01), pages 1559-1574, XP011557475, ISSN: 0885-3010, DOI: 10.1109/TUFFC.2014.3070 [extrait le 2014-08-25]**
• **CHAOLONG SONG ET AL: "Liquid acoustic lens for photoacoustic tomography", OPTICS LETTERS, OPTICAL SOCIETY OF AMERICA, US, vol. 38, no. 15, 1 août 2013 (2013-08-01), pages 2930-2933, XP001583787, ISSN: 0146-9592, DOI: HTTP://DX.DOI.ORG/10.1364/OL.38.002930**
• **CHAN ARTHUR H ET AL: "An image-guided high intensity focused ultrasound device for uterine fibroids treatment", MEDICAL PHYSICS, AIP, MELVILLE, NY, US, vol. 29, no. 11, 1 novembre 2002 (2002-11-01), pages 2611-2620, XP012011657, ISSN: 0094-2405, DOI: 10.1118/1.1513990**

- MARECHAL ET AL: "Lens-focused transducer modeling using an extended KLM model", ULTRASONICS, IPC SCIENCE AND TECHNOLOGY PRESS LTD. GUILDFORD, GB, vol. 46, no. 2, 29 avril 2007 (2007-04-29) , pages 155-167, XP022051608, ISSN: 0041-624X, DOI: 10.1016/J.ULTRAS.2007.01.006

- ÖZÇAM A EVREN ET AL: "Effect of ultraviolet/ozone treatment on the surface and bulk properties of poly(dimethyl siloxane) and poly(vinylmethyl siloxane) networks", POLYMER, vol. 55, no. 14, 17 mai 2014 (2014-05-17), pages 3107-3119, XP028866024, ISSN: 0032-3861, DOI: 10.1016/J.POLYMER.2014.05.027

**Description**

La présente invention est relative aux procédés d'insonification pour obtenir un champ prédéterminé d'ondes ultrasonores dans un milieu sensiblement homogène masqué par une barrière osseuse, et aux procédés de calcul et de fabrication pour réaliser des lentilles ultrasonores à ces fins.

Plus particulièrement, l'invention concerne un procédé d'insonification pour obtenir, à partir d'une sonde ultrasonore, un champ objectif prédéterminé d'ondes ultrasonores dans un milieu comprenant une partie interne sensiblement homogène masquée par au moins une barrière aberratrice, le procédé comprenant une étape de simulation au cours de laquelle on simule au moins une propagation d'ondes ultrasonores dans le milieu à partir d'une cartographie de propriétés de propagation des ondes ultrasonores dans le milieu.

On entend ici par « partie sensiblement homogène » un milieu ayant des caractéristiques sensiblement homogènes pour la propagation des ondes ultrasonores. Il peut s'agir par exemple du cerveau d'un humain ou animal, auquel cas la barrière aberratrice est constituée par le crâne.

Le document WO2004019784A1 décrit un exemple d'un tel procédé, dans lequel, à partir de la simulation susmentionnée, on détermine des signaux individuels à émettre par chaque transducteur pour obtenir le champ objectif dans la partie interne du milieu. Ce procédé fonctionne bien mais nécessite en pratique un grand nombre de transducteurs (typiquement 500 à 1000) et une électronique complexe capable de piloter individuellement chaque transducteur.

Le document Y. Kim et al.: "Rapid prototyping fabrication of focused ultrasound transducers", 2014, décrit une lentille acoustique réalisée par prototypage rapide et utilisable pour le traitement thérapeutique du cerveau par émission focalisée d'ondes ultrasonores. Le dispositif de traitement comporte une pluralité de transducteurs et une lentille acoustique par transducteur.

La présente invention a notamment pour but de perfectionner encore ce type de procédé, notamment afin de permettre sa mise en œuvre par des moyens simplifiés et peu coûteux.

A cet effet, l'invention propose un procédé de calcul d'une lentille ultrasonore selon la revendication 1. Par ailleurs, l'invention a également pour objet un dispositif de calcul d'une lentille ultrasonore selon la revendication 20.

Grâce à ces dispositions, on peut obtenir le champ objectif d'ondes ultrasonores de façon très simple, éventuellement avec un nombre réduit de transducteurs (éventuellement un seul). En particulier, une sonde ultrasonore comprenant une lentille calculée selon l'invention ne requiert pas l'émission d'une forme d'onde particulièrement complexe à partir de la sonde ultrasonore, puisque la sonde peut émettre une onde prédéterminée particulièrement simple, par exemple une onde focalisée à une distance prédéterminée.

Dans des modes de réalisation préférés du procédé de calcul et du dispositif de calcul selon l'invention, on peut éventuellement avoir recours en outre à l'une et/ou à l'autre des dispositions selon les revendications dépendantes.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue d'ensemble schématique d'un exemple dispositif de génération d'ondes ultrasonores mettant en œuvre l'invention, comprenant une lentille ultrasonore,
- la figure 2 est un schéma fonctionnel du dispositif de la figure 1,
- la figure 3 est un schéma illustrant le mode de calcul de la lentille acoustique utilisable dans le dispositif de la figure 1,
- et la figure 4 est un schéma illustrant le fonctionnement du dispositif de la figure 1.

Sur les différentes figures, les mêmes références désignent des éléments identiques ou similaires.

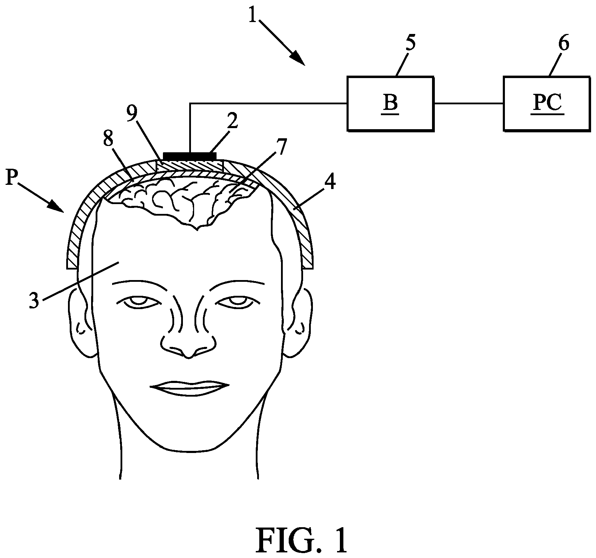

Le dispositif 1 de génération d'ondes ultrasonores représenté sur la figure 1 est adapté pour générer des champs d'ondes ultrasonores prédéterminés dans un milieu 3, par exemple la tête d'un patient P, ou une autre partie du corps. Le milieu 3 comporte au moins une barrière aberratrice 8 (ici le crâne du patient P) et une partie interne sensiblement homogène 7 (ici le cerveau du patient P) masquée par ladite barrière aberratrice 8.

Plus généralement, le milieu sensiblement homogène 7 pourrait être tout milieu tissulaire d'un patient humain ou de tout autre vertébré, milieu tissulaire ayant des caractéristiques sensiblement homogènes pour la propagation des ondes ultrasonores, tandis que la barrière aberratrice pourrait être toute autre barrière osseuse. A titre d'exemple alternatif, le milieu tissulaire pourrait éventuellement être constitué par le cœur ou l'ensemble cœur-poumons du patient P, auquel cas la barrière osseuse serait constituée par la cage thoracique.

Le dispositif 1 est destiné à générer des ondes ultrasonores dans le cerveau 7 du patient P (ou plus généralement dans la partie interne 7 du milieu 3), à des fréquences par exemple de l'ordre de 0,1 à 10 MHz, notamment de 0,2 à 3 MHz, depuis l'extérieur du crâne 8.

Cette génération d'ondes ultrasonores peut être destinée par exemple :

- à réaliser une image ou une série d'images échographiques du cerveau, qu'il s'agisse d'imagerie statique ou fonctionnelle, notamment d'imagerie Doppler permettant de visualiser les écoulements sanguins ou d'imagerie thermique permettant de visualiser les échauffements provoqués par un traitement par hyperthermie,

- et/ou de réaliser un traitement par hyperthermie, notamment pour détruire des tumeurs, coaguler des hémorragies, activer localement des médicaments thermo-activables,
- et/ou ouvrir localement la barrière hémato-encéphalique afin de faire diffuser localement un médicament précédemment injecté par voie intraveineuse
- et/ou activer/inhiber le cerveau par neuromodulation ultrasonore.

Dans ces dernières applications, on notera que l'obtention d'un champ d'ondes objectif n'est jamais en soi un traitement thérapeutique, mais une simple mesure technique de focalisation d'ondes. Le traitement thérapeutique éventuel, décidé par un médecin, consiste dans le choix du champ d'onde objectif, son intensité, sa durée d'application, le nombre d'applications de ce champ d'ondes objectif et leur répartition dans le temps.

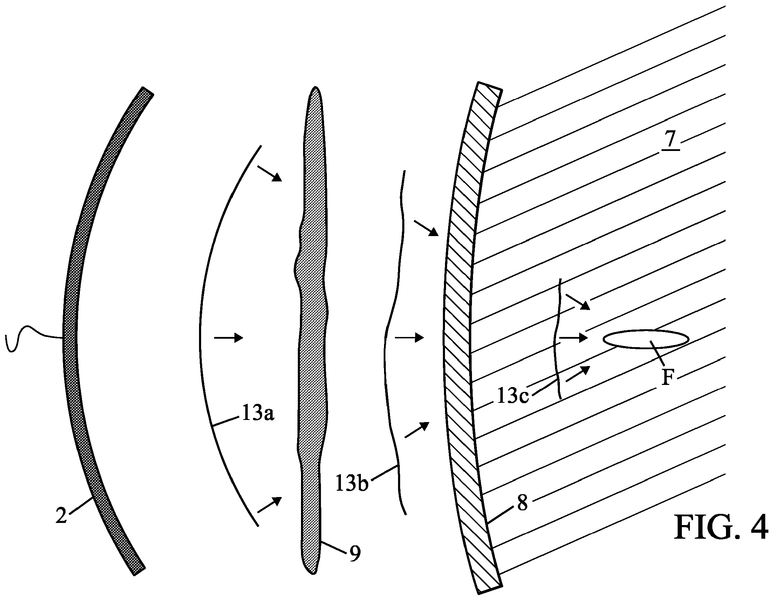

Dans tous les cas, il est nécessaire de pouvoir générer, avec le plus de précision possible, un ou plusieurs champ(s) objectif(s) prédéterminé(s) d'ondes ultrasonores dans le cerveau 7 du patient P, par exemple pour focaliser en un ou plusieurs points du cerveau les ondes ultrasonores émises par une sonde ultrasonore 2, ou pour générer des champs d'ondes plus complexes. Comme représenté sur la figure 4, le champ d'ondes objectif peut par exemple être un champ d'ondes focalisé en une tache focale F dans le cerveau 7, ou éventuellement plusieurs taches focales.

Comme représenté sur la figure 1, la sonde ultrasonore 2 peut être par exemple intégrée dans un casque 4 positionné de façon prédéterminée sur la tête 3 du patient P, ou bien ladite sonde ultrasonore 2 peut être portée par tout autre système de positionnement connu. Dans tous les cas, la sonde ultrasonore 2 est positionnée à l'extérieur du crâne 8 du patient dans une position prédéterminée.

Une lentille acoustique 9, dite ici également lentille ultrasonore, également intégrée au casque 4, est interposée entre la sonde ultrasonore 2 et le crâne 8 du patient P. Un gel ou un liquide, éventuellement contenu dans une poche souple (non représentée), peut être interposé entre la lentille ultrasonore 9 et le crâne 8 et/ou entre la sonde ultrasonore 2 et la lentille ultrasonore 9, de façon à assurer une bonne transmission des ondes ultrasonores. Ce gel ou liquide sera ici appelé milieu externe et considéré comme faisant partie du milieu de propagation 3 des ondes ultrasonores.

La lentille acoustique 9 peut être réalisée en tout matériau dans lequel la célérité $c_l$ des ondes acoustiques de compression est différente de la célérité c desdites ondes dans le cerveau (c est sensiblement la célérité du son dans l'eau, soit environ 1480 m/s). La lentille ultrasonore peut être réalisée par exemple en matière plastique, notamment en polydiméthylsiloxane également appelé PDMS ($c_l$=1030 m/s) ou en polyméthylpentène, connu sous l'appellation TPX® ($c_l$=2090 m/s).

Eventuellement, certaines parties de la lentille ultrasonore peuvent être réalisées en un matériau atténuant, réfléchissant ou absorbant les ondes ultrasonores, notamment pour atténuer localement le champ d'ondes ultrasonore produit dans le cerveau 7 ou le crâne 8, par exemple dans les zones ne nécessitant pas de traitement ou pour limiter l'échauffement du crâne.

La sonde ultrasonore 2 peut être commandée par un circuit électronique 5 (B) lui-même éventuellement contrôlé par un ordinateur 6 (PC).

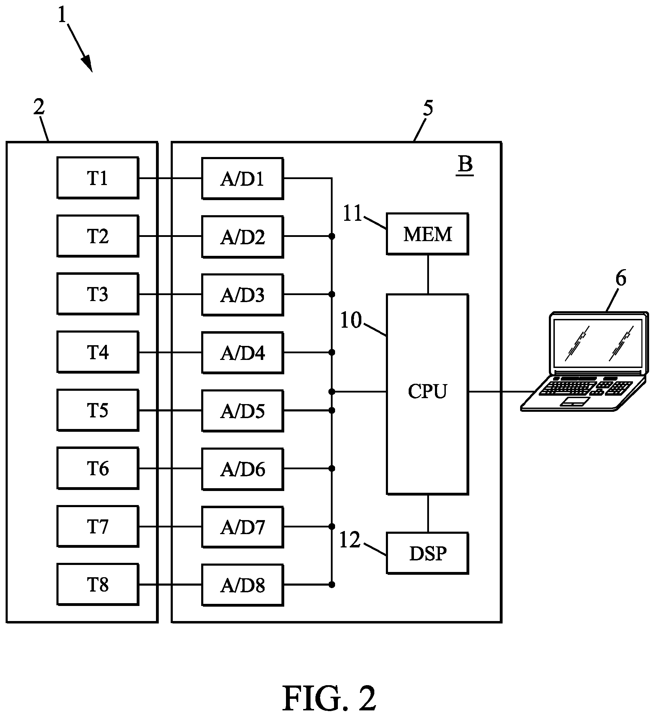

Comme représenté sur la figure 2, la sonde ultrasonore 2 peut comprendre un ou plusieurs transducteur(s) ultrasonores T1-T8. Ces transducteurs sont au nombre de 8 dans l'exemple représenté, mais pourraient être plus nombreux notamment dans des applications d'imagerie, ou moins nombreux. Eventuellement, on peut utiliser un seul transducteur T1, notamment lorsqu'on cherche uniquement à focaliser l'onde ultrasonore notamment aux fins de traitement par effet thermique ou mécanique. La sonde ultrasonore 2 peut éventuellement comporter des transducteurs destinés à l'imagerie ultrasonore et d'autres destinés au traitement par hyperthermie, comme expliqué dans le document WO2004019784A1 susmentionné.

Comme représenté sur la figure 2, le circuit électronique 5 qui commande la sonde ultrasonore 2 peut comprendre :

- une unité centrale électronique 10 (CPU) commandée par l'ordinateur 7,
- au moins une mémoire centrale 11 (MEM) reliée à l'unité centrale électronique 10,
- un convertisseur analogique-digital A/D1-A/D8 relié à chaque transducteur T1-T8 et généralement associé à une mémoire tampon (non représentée),
- éventuellement, un circuit spécifique de traitement de signal 12 (DSP).

Pour mettre en œuvre le dispositif décrit précédemment, on conçoit et fabrique la lentille ultrasonore 9 de façon qu'elle permette d'obtenir le champ d'ondes objectif souhaité, par exemple en suivant les étapes suivantes :

**(a) Détermination d'un modèle tridimensionnel du milieu 3** :

On détermine préalablement un modèle tridimensionnel du milieu 3 comprenant une cartographie de propriétés de propagation des ondes ultrasonores dans le milieu.

Cette étape comprend généralement une opération d'imagerie, notamment imagerie rayons X ou IRM, notamment afin de déterminer la densité osseuse de la barrière aberratrice 8 en chaque point, ce qui permet de déterminer les paramètres de propagation des ondes ultrasonores en chaque point, comme expliqué dans le document WO2004019784A1 susmentionné (notamment densité massique $\rho$, célérité c des ondes ultrasonores et/ou absorption $\tau$ desdites ondes ultrasonores, en tout point de la barrière aberratrice 8).

Le modèle tridimensionnel peut être par exemple chargé dans l'ordinateur 6. Un utilisateur peut positionner sur l'image du milieu 3 le champ d'onde ultrasonore souhaité, par exemple la ou les taches focale(s) F souhaitée(s) (Figure 4). Ce positionnement peut s'effectuer par exemple en visualisant sur l'image du milieu 3 une lésion à traiter ou autre zone d'intérêt, et en repérant cette zone sur l'image à l'aide de l'interface utilisateur de l'ordinateur 6 (par exemple souris, écran tactile ou autre). Dans ce qui suit, on fera référence à « la zone d'intérêt F » par mesure de simplicité, pour désigner une ou plusieurs taches focales ou une ou des zones à champ d'onde objectif plus complexe.

**(b) simulation** :

A partir du modèle tridimensionnel susmentionné, on peut simuler la propagation d'une onde ultrasonore à travers la barrière aberratrice pour ensuite déterminer les délais ou déphasages qui devront être induits par la lentille, puis en déduire la forme à donner à la lentille. Cette étape peut être réalisée par l'ordinateur 6 susmentionné ou un autre ordinateur.

Cette étape de simulation peut comporter les sous-étapes suivantes :

**(b1) Détermination des temps d'arrivée sur une surface de contrôle (S1 - S'1)** :

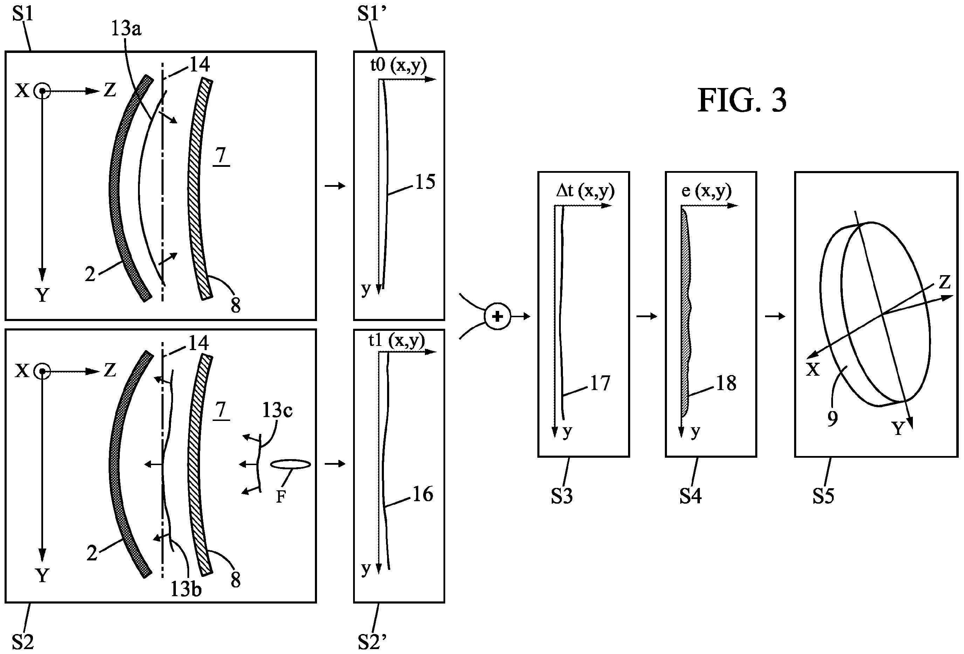

Comme représenté en S1 et S'1 sur la figure 3, il est possible de déterminer des temps d'arrivée $t_0(x,y)$ d'une onde ultrasonore prédéterminée 13 émise par la sonde ultrasonore 2, en différents points de contrôle M(x,y), appartenant préférentiellement à une même surface de contrôle 14 disposée entre la sonde 2 et la zone d'intérêt F susmentionnée. Cette surface de contrôle 14 est située entre la barrière aberratrice 8 et la sonde ultrasonore 2, à la position qui doit être occupée par la lentille acoustique 9 susmentionnée. La surface de contrôle 14 peut par exemple être un plan XY dont les axes X, Y sont perpendiculaires à l'axe Z disposé dans le sens de la profondeur. Cette surface de contrôle pourrait être toute autre surface, plane ou courbe.

Ces temps d'arrivée $t_0(x,y)$ sont aisément déterminés par l'ordinateur 6 ou autre (à une constante de temps $T'_0$ près quelconque commune à toute la surface de contrôle 14), puisque la partie externe du milieu (à l'extérieur de la barrière aberratrice) est considérée comme homogène pour la propagation des ondes ultrasonores.

Dans le cas simple et le plus courant où la sonde est sphérique de rayon R, cette détermination peut être faite très simplement sous la forme :

$$t_0(x,y) = T'_0 - d(M)/c \quad (2)$$

où :

- d(M) est la distance entre le point de contrôle M(x,y) et le centre de courbure de la sonde,
- c est la célérité de l'onde ultrasonore dans la partie externe du milieu 3.

Plus généralement, les temps d'arrivée $t_0(x,y)$ peuvent être déterminés par calcul de propagation de l'onde ultrasonore entre la sonde et les points de contrôle ou par toute méthode connue de propagation d'onde, ce qui est simple en milieu homogène (on peut alors effectuer une propagation dans un modèle simplifié permettant un calcul analytique).

Les temps d'arrivée $t_0(x,y)$ seront dits ci-après premiers temps d'arrivée.

**(b2) simulation de la rétro-propagation d'onde ultrasonore voulue dans la zone d'intérêt dans le modèle tridimensionnel (S2):**

Comme représenté en S2 sur la figure 3, au cours de cette sous-étape b2, on simule une rétro-propagation du champ objectif prédéterminé d'onde ultrasonore dans le milieu 3, depuis la zone d'intérêt F dans le milieu 3 jusqu'à la surface de contrôle 14 susmentionnée.

Cette simulation peut être effectuée par exemple par l'ordinateur 6, par exemple en utilisant une équation d'ondes telle que l'équation (1) ci-après :

$$(1+\tau\ (\vec{r})\frac{\partial}{\partial t}\cdot)\left[\rho\ (\vec{r})\nabla\cdot\left(\frac{1}{\rho\ (\vec{r})}\nabla p(\vec{r},t)\right)\right]-\frac{1}{c\ (\vec{r})^2}\frac{\partial^2 p(\vec{r},t)}{\partial t^2}=S\ (\vec{r},t)\quad (1)\ ,$$

où $\vec{r}$ désigne un vecteur de position du point considéré, p désigne la pression et S désigne les signaux ultrasonores générés par une source ultrasonore éventuellement présente au point considéré.

La propagation des ondes ultrasonores dans la barrière aberratrice 8 et la partie interne 7 peut être simulée dans l'ordinateur par différences finies, en discrétisant l'équation (1) ci-dessus. Cette simulation peut également être effectuée par éléments finis, ou par une méthode de diffraction impulsionnelle, ou toute autre méthode connue.

**(b3) Détermination des deuxièmes temps d'arrivée sur la surface de contrôle 14 (S'2)** :

Comme représenté en S'2 sur la figure 3, la simulation susmentionnée permet de déterminer des temps d'arrivée $t_1(x,y)$ de l'onde ultrasonore 13c, 13b représentative de la rétro-propagation du champ d'onde objectif prédéterminé auxdits points de contrôle M(x,y) appartenant à la surface de contrôle 14.

Les temps d'arrivée $t_1(x,y)$ sont comptés par exemple à partir de l'instant d'émission de l'onde ultrasonore 13c dans ladite zone F, x et y étant respectivement les coordonnées de chaque point de contrôle M considéré, sur les axes X, Y. Les temps d'arrivée $t_1(x,y)$ sont dits ci-après deuxièmes temps d'arrivée.

**(b4) Détermination de la loi de délai de la lentille (S3)** :

Comme représenté en S3 sur la figure 3, on détermine une loi de délai $\Delta t(x,y)$ ou loi de retard auxdits différents points de contrôle M(x,y), égale à la somme des premiers temps d'arrivée $t_0(x,y)$ et des deuxièmes temps d'arrivée $t_1(x,y)$.

**(c)Calcul de la lentille ultrasonore (S4)** :

A partir de la loi de délai susmentionnés $\Delta t(x,y)$, l'ordinateur 6 ou autre calcule ensuite le profil de la lentille ultrasonore 9 qui, lorsqu'elle est interposée entre la sonde ultrasonore 2 et la barrière aberratrice 8, est adaptée pour introduire ledit délai $\Delta t(x,y)$, de façon que lorsque la sonde ultrasonore 2 émet ladite onde ultrasonore prédéterminée 13a, ladite onde prédéterminée produit après traversée de la lentille puis de la barrière aberratrice le champ d'onde ultrasonore prédéterminé dans la zone d'intérêt .

Plus précisément, on peut calculer une épaisseur e de la lentille ultrasonore 9, en chaque point de ladite lentille correspondant à un point de contrôle M(x,y) de la surface de contrôle 14, pour que l'épaisseur e de lentille introduise une correction de temps de propagation d'onde ultrasonore correspondant à ladite loi de délai, modulo la période T=1/f de l'onde ultrasonore (f est la fréquence centrale de l'onde ultrasonore) et / ou à une constante de temps $T_0$ près quelconque commune à toute la surface de contrôle.

Par exemple, on peut calculer l'épaisseur e(x,y) de lentille ultrasonore, en chaque point L(x,y) de la lentille correspondant à un point de contrôle M(x,y), par la formule (3) suivante :

$$e(x,y)=\Delta t(x,y)/(1/c-1/c_1)+e_0 \quad\quad (3).$$

La constante $e_0$ est un nombre réel quelconque, tel que e(x,y) ait une valeur positive et minimale en tout point de la lentille.

Dans le cas de l'émission d'une onde monochromatique, on peut calculer l'épaisseur e(x,y) de lentille ultrasonore, en chaque point L(x,y) de la lentille correspondant à un point de contrôle M(x,y), par la formule (4) suivante :

$$e(x,y)=\Delta t(x,y)/(1/c-1/c_1)+e_0,\ \text{modulo}\ T/(1/c-1/c_1) \quad\quad (4).$$

Modulo $T/(1/c-1/c_1)$ signifie que e(x,y)=$\Delta t(x,y)/(1/c-1/c_1)+e_0+n.T/(1/c-1/c_1)$, où n est un entier positif ou négatif tel que e(x ,y) ait une valeur positive. L'entier n peut être commun à toute la lentille 9 (lentille simple) ou éventuellement être différent suivant les régions de la lentille 9 (lentille de Fresnel).

On notera que l'étape (c) de calcul de la lentille peut tenir compte au moins des temps de parcours de l'onde ultrasonore prédéterminée dans la lentille ultrasonore 9 et des angles d'incidences de ladite onde ultrasonore prédéterminée à la surface de la lentille ultrasonore 9. L'étape (c) de calcul de lentille peut aussi tenir compte de l'ensemble de la propagation de ladite onde ultrasonore prédéterminée, y compris des réverbérations entre la sonde ultrasonore 2, la lentille ultrasonore 9 et la barrière aberratrice 8. Eventuellement l'étape (c) de calcul de lentille peut se faire itérativement afin d'optimiser le rendement de la transmission d'énergie ultrasonore dans la zone prédéterminée F, à l'aide d'un modèle analytique ou numérique.

**(d) Réalisation de la lentille ultrasonore (S5)** :

Une fois calculé le profil de la lentille 9 pour le patient P (ou plus généralement pour une application donnée), celle-ci peut être fabriquée, de préférence par une méthode de fabrication rapide.

On peut en particulier utiliser des méthodes de fabrication couramment utilisées en prototypage rapide, par exemple :

- par impression tridimensionnelle de la lentille 9,
- par fraisage à commande numérique d'un bloc de matériau pour former la lentille 9,
- par un procédé en deux sous-étapes comprenant :

    (d1) une sous-étape de réalisation de moule (non représenté) effectuée par une méthode choisie parmi l'impression tridimensionnelle du moule et le fraisage à commande numérique d'un bloc de matériau pour former le moule,
    (d2) une sous-étape de moulage dans laquelle on moule ladite lentille 9 dans ledit moule.

**(e)Utilisation** :

Une fois la lentille 9 réalisée, on peut la fixer dans le casque 4 susmentionné ou autre support, et ainsi la positionner dans la position prédéterminée qu'elle doit occuper sur le crâne 8 du patient P. Un opérateur peut alors faire émettre l'onde prédéterminée 13a par la sonde ultrasonore 2 au travers de la lentille ultrasonore 9.

Comme représenté sur la figure 4, la lentille 9 introduit des aberrations qui déforment le front d'onde 13b en amont du crâne 8, de façon qu'après passage au travers du crâne 8 ou autre barrière aberratrice, le front d'onde 13c corresponde à l'onde théorique 13 susmentionnée. On obtient ainsi le champ d'ondes objectif, par exemple une focalisation dans la tache focale F.

L'onde ainsi émise dans le cerveau ou autre partie interne 7 peut servir à traiter la zone d'intérêt F comme expliqué précédemment. De plus, on peut éventuellement imager le cerveau au ou autre partie interne 7 au moins au voisinage de la zone d'intérêt F, lorsque la sonde ultrasonore 2 comporte des transducteurs ultrasonore d'imagerie, puisque la lentille ultrasonore 9 compense les aberrations dues au crâne ou autre barrière aberratrice 8, aussi bien en émission qu'en réception.

Le procédé selon l'invention peut en outre comporter une étape facultative (f) de vérification du positionnement du dispositif, au cours de laquelle on enregistre sur la sonde ultrasonore les échos ultrasonores qui se sont réfléchis sur ladite barrière aberratrice et on les compare aux mêmes signaux réfléchis simulés en utilisant le modèle susmentionné du milieu 3.

Dans tous les cas de figures, la lentille 9 peut être réalisée en un matériau ayant des caractéristiques mécaniques (notamment la dureté), et donc des caractéristiques de propagation d'ondes acoustiques, modifiables par exposition à un rayonnement prédéterminé.

Le rayonnement prédéterminé peut par exemple être un rayonnement ultra-violet.

Le matériau en question peut par exemple être le poly dimethyl siloxane (PDMS) ou le poly vinylmethyl siloxane (PVMS), durcissables au rayonnement ultra-violet.

Dans ce cas, au cours de l'étape (c) de calcul de lentille, on peut calculer calcule la lentille 9 en déterminant des caractéristiques mécaniques locales de propagation d'ondes acoustiques dans ladite lentille 9, qui permettent d'atteindre le champ d'onde objectif souhaité. Ces caractéristiques mécaniques peuvent être déterminées en plus de la forme de la lentille 9, ou bien en variante, le champ d'onde objectif pourrait être obtenues par les seules variations locales de caractéristiques locales de propagation dans la lentille 9.

Au cours de l'étape (d) de réalisation de la lentille 9, on expose localement ladite lentille audit rayonnement prédéterminé pour obtenir les caractéristiques mécaniques locales de propagation d'ondes acoustiques déterminées à l'étape (c) de calcul de lentille.

**Revendications**

1. Procédé, mis en œuvre par ordinateur, de calcul d'une lentille ultrasonore adaptée pour une insonification d'un milieu comprenant au moins une barrière aberratrice (8) et une partie interne (7) sensiblement homogène masquée par ladite barrière aberratrice (8),

   le procédé de calcul comprenant au moins une étape de calcul de lentille (c) au cours de laquelle on calcule, en utilisant un modèle du milieu comprenant une cartographie de propriétés de propagation des ondes ultrasonores obtenue à partir d'une imagerie réelle du milieu (3), une lentille ultrasonore (9), la lentille ultrasonore (9) étant configurée pour, lorsque la lentille ultrasonore (9) est interposée entre une sonde ultrasonore (2) et la barrière aberratrice (8) et lorsque la sonde ultrasonore (2) placée dans une position prédéterminée par rapport audit milieu (3) à l'opposé de ladite partie interne (7) par rapport à ladite barrière aberratrice (8) émet une onde ultrasonore prédéterminée (13a), générer des aberrations telles que ladite onde prédéterminée génère un champ objectif prédéterminé d'onde ultrasonore dans au moins une zone prédéterminée (F) appartenant à ladite partie interne (7).

2. Procédé de fabrication pour réaliser la lentille calculée selon le procédé de la revendication 1, comportant en outre une étape (d) de réalisation de la lentille (9) calculée à l'étape (c) de calcul de lentille.

3. Procédé de fabrication selon la revendication 2, dans lequel l'étape (d) de réalisation de la lentille (9) est effectuée par une méthode choisie parmi l'impression tridimensionnelle de la lentille (9) et le fraisage à commande numérique d'au moins un bloc de matériau pour former la lentille (9).

4. Procédé de fabrication selon la revendication 2, dans lequel l'étape (d) de réalisation de la lentille (9) comprend les sous-étapes suivantes :

   (d1) une sous-étape de réalisation de moule effectuée par une méthode choisie parmi l'impression tridimensionnelle dudit au moins un moule et le fraisage à commande numérique d'au moins un bloc de matériau pour former ledit au moins un moule,
   (d2) une sous-étape de moulage dans laquelle on moule ladite lentille (9) ou au moins un composant de ladite lentille (9) dans ledit au moins un moule.

5. Procédé selon l'une quelconque des revendications 1 à 4, dans lequel l'étape (c) de calcul de lentille est précédée par une étape d'imagerie (a) au cours de laquelle est réalisée ladite cartographie.

6. Procédé selon la revendication 5, dans lequel l'étape d'imagerie (a) est réalisée par imagerie rayons X ou par IRM.

7. Procédé selon la revendication 6, dans lequel ladite cartographie est réalisée à partir de mesures de densité osseuse.

8. Procédé selon l'une quelconque des revendications 1 à 7, dans lequel l'étape (c) de calcul de lentille est précédée par une étape de simulation (b) au cours de laquelle :

   (b1) on calcule une propagation d'une onde ultrasonore prédéterminée (13a) dans le milieu depuis la sonde ultrasonore (2) disposée à ladite position prédéterminée, jusqu'à une position choisie de la lentille (9) et on détermine des premiers temps d'arrivée $t_0(x,y)$ de ladite onde ultrasonore prédéterminée au niveau de ladite position choisie de la lentille (9),
   (b2) on simule une rétro-propagation dudit champ objectif prédéterminé d'onde ultrasonore dans le milieu (3), depuis la zone prédéterminée (F) jusqu'à ladite position choisie de la lentille (9),
   (b3) on détermine des deuxième temps d'arrivée $t_1(x,y)$ de ladite rétro-propagation dudit champ objectif prédéterminé d'onde ultrasonore à ladite position choisie de la lentille (9),
   (b4) on détermine une loi de délai $\Delta t(x,y)$ à ladite position choisie de la lentille (9), égale à la somme des premiers temps d'arrivée $t_0(x,y)$ et des deuxièmes temps d'arrivée $t_1(x,y)$,

   et au cours de l'étape (c) de calcul de lentille, on calcule la lentille ultrasonore (9) à partir de ladite loi de délai $\Delta t(x,y)$, de façon que lorsque la sonde ultrasonore (2) émet ladite onde ultrasonore prédéterminée (13a) au travers de ladite lentille ultrasonore, ladite onde prédéterminée reproduise le champ d'ondes objectif dans ladite zone F, après traversé de la barrière aberratrice (8).

9. Procédé selon la revendication 8, dans lequel les premiers temps d'arrivée $t_0(x,y)$ et les deuxièmes temps d'arrivée $t_1(x,y)$ sont déterminés en différents points de contrôle M(x,y) appartenant à une surface de contrôle prédéterminée (14) située à ladite position choisie de la lentille (9).

10. Procédé selon la revendication 9, dans lequel la surface de contrôle (14) est située entre la sonde ultrasonore (2) et la barrière aberratrice (8).

11. Procédé selon l'une quelconque des revendications 9 et 10, dans lequel à l'étape (c) de calcul de lentille (9), on calcule une épaisseur e(x,y) de lentille ultrasonore, en chaque point L(x,y) de la lentille correspondant à un point de contrôle M(x,y), par la formule suivante :

$$e(x,y)= \Delta t(x,y)/(1/c-1/c_1)+ e_0$$

où :

- c est la célérité de l'onde ultrasonore dans le milieu (3) hors de la barrière aberratrice (8),
- $c_1$ est la célérité de l'onde ultrasonore dans la lentille ;
- $e_0$ est un nombre réel tel que e(x,y) ait une valeur positive et supérieure à une épaisseur prédéterminée en tout point de la lentille.

12. Procédé selon l'une quelconque des revendications 9 et 10, dans lequel l'onde ultrasonore est monochromatique et à l'étape (c) de calcul de lentille (9), on calcule une épaisseur e(x,y) de lentille, en chaque point L(x,y) de la lentille correspondant à un point de contrôle M(x,y), par la formule suivante :

$$e(x,y)= \Delta t(x,y)/(1/c-1/c_1)+ e_0, modulo\ T/(1/c-1/c_1).$$

où :

- c est la célérité de l'onde ultrasonore dans le milieu hors de la barrière aberratrice,
- $c_1$ est la célérité de l'onde ultrasonore dans la lentille ;
- $e_0$ est un nombre réel tel que e(x,y) ait une valeur positive et supérieure à une épaisseur prédéterminée en tout point de la lentille,
- T est la période de l'onde ultrasonore.

13. Procédé selon l'une quelconque des revendications 8 à 12, dans lequel à la sous-étape (b3), on détermine les premiers temps d'arrivée $t_0(x,y)$ en simulant une propagation de ladite onde ultrasonore prédéterminée dans un modèle simplifié permettant un calcul analytique.

14. Procédé selon l'une quelconque des revendications 1 à 13, dans lequel l'étape (c) de calcul de la lentille tient compte des temps de parcours de ladite onde ultrasonore prédéterminée dans la lentille ultrasonore (9) et des angles d'incidences de ladite onde ultrasonore prédéterminée à la surface de la lentille ultrasonore (9).

15. Procédé selon l'une quelconque des revendications 1 à 14, dans lequel l'étape (c) de calcul de lentille tient compte de l'ensemble de la propagation de ladite onde ultrasonore prédéterminée, y compris des réverbérations entre la sonde ultrasonore (2), la lentille ultrasonore (9) et la barrière aberratrice (8).

16. Procédé selon l'une quelconque des revendications 1 à 15, dans lequel l'étape (c) de calcul de lentille se fait itérativement afin d'optimiser le rendement de la transmission d'énergie ultrasonore dans la zone prédéterminée (F), à l'aide d'un modèle analytique ou numérique.

17. Procédé selon l'une quelconque des revendications 1 à 16, dans lequel la lentille (9) est réalisée en un matériau ayant des caractéristiques de propagation d'ondes acoustiques modifiables par exposition à un rayonnement prédéterminé,

au cours de l'étape (c) de calcul de lentille, on calcule la lentille en déterminant des caractéristiques mécaniques locales de propagation d'ondes acoustiques dans ladite lentille (9),

et le procédé comporte une étape (d) de réalisation de la lentille (9) au cours de laquelle on expose localement ladite lentille audit rayonnement prédéterminé pour obtenir les caractéristiques mécaniques locales de propagation d'ondes acoustiques déterminées à l'étape (c) de calcul de lentille.

**18.** Procédé d'insonification d'un milieu (3) comprenant au moins une barrière aberratrice (8) et une partie interne sensiblement homogène (7) masquée par ladite barrière aberratrice (8),

le procédé d'insonification comprenant les étapes d'un procédé de fabrication d'une lentille ultrasonore (9) selon l'une quelconque des revendications 2 - 17;
le procédé d'insonification comprenant en outre une étape d'émission au cours de laquelle on fait émettre ladite onde ultrasonore prédéterminée (13a) au travers de la lentille ultrasonore (9) par une onde ultrasonore placée dans ladite position prédéterminée,
ladite onde prédéterminée générant ledit champ objectif prédéterminé d'onde ultrasonore en ladite au moins une zone prédéterminée (F) appartenant à ladite partie interne (7).

**19.** Procédé d'insonification selon la revendication 18, dans lequel ledit champ prédéterminé d'onde ultrasonore est focalisé dans ladite au moins une zone prédéterminée (F).

**20.** Dispositif de calcul d'une lentille ultrasonore (9),

la lentille ultrasonore étant adaptée pour une insonification d'un milieu comprenant au moins une barrière aberratrice (8) et une partie interne (7) sensiblement homogène masquée par ladite barrière aberratrice (8),
le dispositif de calcul comprenant des moyens de calcul de lentille (c) configurés pour calculer ladite lentille ultrasonore (9) en utilisant un modèle du milieu comprenant une cartographie de propriétés de propagation des ondes ultrasonores obtenue à partir d'une imagerie réelle du milieu (3),
ladite lentille ultrasonore (9) étant configurée pour, lorsqu'elle est interposée entre une sonde ultrasonore (2) et la barrière aberratrice (8) et lorsque la sonde ultrasonore (2) placée dans une position prédéterminée par rapport audit milieu (3) à l'opposé de ladite partie interne (7) par rapport à ladite barrière aberratrice (8) émet une onde ultrasonore prédéterminée (13a), générer des aberrations telles que ladite onde prédéterminée génère un champ objectif prédéterminé d'onde ultrasonore en au moins une zone prédéterminée (F) appartenant à ladite partie interne (7).

**21.** Dispositif de fabrication d'une lentille ultrasonore, comprenant un dispositif de calcul d'une lentille ultrasonore selon la revendication 20 et des moyens de réalisation de la lentille ultrasonore (9).

## Patentansprüche

**1.** Computer-implementiertes Verfahren zum Berechnen einer Ultraschalllinse, welche zur Beschallung eines Milieus geeignet ist, das mindestens eine aberrierende Barriere (8) und einen im Wesentlichen homogenen inneren Teil (7) aufweist, der von der aberrierenden Barriere (8) verdeckt ist,

wobei das Berechnungsverfahren mindestens einen Schritt des Berechnens einer Linse (c) aufweist, in dessen Verlauf eine Ultraschalllinse (9) unter Verwendung eines Modells des Milieus berechnet wird, welches eine anhand eine realen Abbildung des Milieus (3) erhaltene Kartierung der Ausbreitungseigenschaften der Ultraschallwellen aufweist,
wobei die Ultraschalllinse (9) dazu ausgebildet ist, wenn die Ultraschalllinse (9) zwischen einer Ultraschallsonde (2) und der aberrierende Barriere (8) angeordnet ist und wenn die Ultraschallsonde (2), welche in Bezug auf das Milieu (3) an einer vorbestimmten Position dem inneren Teil (7) in Bezug auf die aberrierende Barriere (8) gegenüberliegend platziert ist, eine vorbestimmte Ultraschallwelle (13a) emittiert, Aberrationen zu erzeugen, so dass die vorbestimmte Welle ein vorbestimmtes Ziel-Ultraschallwellenfeld in mindestens einer vorbestimmten Zone (F) erzeugt, welche zu dem inneren Teil (7) gehört.

**2.** Herstellungsverfahren zur Bildung der nach dem Verfahren von Anspruch 1 berechneten Linse, ferner mit einem Schritt (d) des Bildens der im Schritt (c) des Berechnens der Linse berechneten Linse (9).

**3.** Herstellungsverfahren nach Anspruch 2, bei welchem der Schritt (d) des Bildens der Linse (9) mittels eines Verfahrens durchgeführt wird, welches unter dem dreidimensionalen Drucken der Linse (9) und dem CNC-Fräsen mindestens eines Materialblocks zur Bildung der Linse (9) gewählt ist.

4. Herstellungsverfahren nach Anspruch 2, bei welchem der Schritt (d) des Bildens der Linse (9) die folgenden Unterschritte aufweist:

(d1) einen Unterschritt der Formherstellung mittels eines Verfahrens, welches unter dem dreidimensionalen Drucken der mindestens einen Form und dem CNC-Fräsen mindestens eines Materialblocks zur Bildung der mindestens einen Form gewählt ist,
(d2) einen Unterschritt des Formens, bei welchem die Linse (9) oder zumindest ein Bestandteil der Linse (9) in der mindestens einen Form geformt wird.

5. Verfahren nach einem der Ansprüche 1 bis 4, bei welchem dem Schritt (c) des Berechnens der Linse ein Bildgebungsschritt (a) vorausgeht, in dessen Verlauf die Kartierung vorgenommen wird.

6. Verfahren nach Anspruch 5, bei welchem der Bildgebungsschritt (a) durch Röntgenbildgebung oder Kernspintomografie durchgeführt wird.

7. Verfahren nach Anspruch 6, bei welchem die Kartierung anhand von Messungen der Knochendichte erfolgt.

8. Verfahren nach einem der Ansprüche 1 bis 7, bei welchem dem Schritt (c) des Berechnens der Linse ein Simulationsschritt (b) vorausgeht, in dessen Verlauf:

(b1) eine Ausbreitung einer vorbestimmten Ultraschallwelle (13a) in dem Milieu von der an der vorbestimmten Position angeordneten Ultraschallsonde (2) bis zu einer gewählten Position der Linse (9) berechnet wird und erste Ankunftszeiten $t_0(x,y)$ der vorbestimmten Ultraschallwelle (13a) an der gewählten Position der Linse (9) bestimmt werden,
(b2) eine Rückwärtsausbreitung des vorbestimmten Ziel-Ultraschallwellenfelds in dem Milieu (3) von der vorbestimmte Zone (F) bis zu der gewählten Position der Linse (9) simuliert wird,
(b3) zweite Ankunftszeiten $t_1(x,y)$ der Rückwärtsausbreitung des vorbestimmten Ziel-Ultraschallwellenfelds an der gewählten Position der Linse (9) bestimmt werden,
(b4) ein Gesetz des Verzugs $\Delta t(x,y)$ an der gewählten Position der Linse (9), das gleich der Summe der ersten Ankunftszeiten $t_0(x,y)$ und der zweiten Ankunftszeiten $t_1(x,y)$ ist, bestimmt wird,

und im Verlauf des Schritts (c) des Berechnens der Linse die Ultraschalllinse (9) ausgehend von dem Gesetz des Verzugs $\Delta t(x,y)$ derart berechnet wird, dass, wenn die Ultraschallsonde (2) die vorbestimmte Ultraschallwelle (13a) durch die Ultraschalllinse emittiert, die vorbestimmte Welle das Ziel-Wellenfeld in der Zone F nach dem Durchgang durch die aberrierende Barriere (8) reproduziert.

9. Verfahren nach Anspruch 8, bei welchem die ersten Ankunftszeiten $t_0(x,y)$ und die zweiten Ankunftszeiten $t_1(x,y)$ an verschiedenen Kontrollpunkten $M(x,y)$ bestimmt werden, die zu einer vorbestimmten Kontrollfläche (14) gehören, welche sich an der gewählten Position der Linse (9) befindet.

10. Verfahren nach Anspruch 9, bei welchem die Kontrollfläche (14) sich zwischen der Ultraschallsonde (2) und der aberrierenden Barriere (8) befindet.

11. Verfahren nach einem der Ansprüche 9 und 10, bei welchem im Schritt (c) des Berechnens der Linse (9) eine Dicke $e(x,y)$ der Ultraschalllinse an jedem Punkt $L(x,y)$ der Linse, der einem Kontrollpunkt $M(x,y)$ entspricht, mittels der folgenden Formel berechnet wird:

$$e(x,y) = \Delta t(x,y)/(1/c - 1c_1) + e_0$$

wobei:

- $c$ die Geschwindigkeit der Ultraschallwelle in dem Milieu (3) außerhalb der aberrierenden Barriere (8) angibt,
- $c_1$ die Geschwindigkeit der Ultraschallwelle in der Linse angibt;
- $e_0$ eine reelle Zahl ist, so dass $e(x,y)$ einen positiven Wert aufweist und größer als eine vorbestimmte Dicke an jedem Punkt der Linse ist.

**12.** Verfahren nach einem der Ansprüche 9 und 10, bei welchem die Ultraschallwelle monochromatisch ist und im Schritt (c) des Berechnens der Linse (9) eine Dicke $e(x,y)$ der Linse an jedem Punkt $L(x,y)$ der Linse, der einem Kontrollpunkt $M(x,y)$ entspricht, mittels der folgenden Formel berechnet wird:

$$e(x,y) = \Delta t(x,y)/(1/c - 1c_1) + e_0, \text{ Modulo } T/(1/c - 1c_1).$$

wobei:

- $c$ die Geschwindigkeit der Ultraschallwelle in dem Milieu außerhalb der aberrierenden Barriere angibt,
- $c_1$ die Geschwindigkeit der Ultraschallwelle in der Linse angibt;
- $e_0$ eine reelle Zahl ist, so dass $e(x,y)$ einen positiven Wert aufweist und größer als eine vorbestimmte Dicke an jedem Punkt der Linse ist;
- $T$ die Periode der Ultraschallwelle angibt.

**13.** Verfahren nach einem der Ansprüche 8 bis 12, bei welchem in dem Unterschritt (b3) die ersten Ankunftszeiten $t_0(x,y)$ bestimmt werden, indem eine Ausbreitung der vorbestimmten Ultraschallwelle in einem vereinfachten Modell simuliert, das eine analytische Berechnung erlaubt.

**14.** Verfahren nach einem der Ansprüche 1 bis 13, bei welchem der Schritt (c) des Berechnens der Linse die Laufzeiten der vorbestimmten Ultraschallwelle in der Ultraschalllinse (9) und die Einfallswinkel der vorbestimmten Ultraschallwelle auf die Oberfläche der Ultraschalllinse (9) berücksichtigt.

**15.** Verfahren nach einem der Ansprüche 1 bis 14, bei welchem der Schritt (c) des Berechnens der Linse die Gesamtheit der Ausbreitung der vorbestimmten Ultraschallwelle berücksichtigt, einschließlich des Nachhalls zwischen der Ultraschallsonde (1), der Ultraschalllinse (9) und der aberrierenden Barriere (8).

**16.** Verfahren nach einem der Ansprüche 1 bis 15, bei welchem der Schritt (c) des Berechnens der Linse iterativ durchgeführt wird, um den Wirkungsgrad der Ultraschallenergieübertragung in der vorbestimmten Zone (F) mit Hilfe eines analytischen oder digitalen Modells zu optimieren,

**17.** Verfahren nach einem der Ansprüche 1 bis 16, bei welchem die Linse (9) aus einem Material gebildet wird, das Schallwellenausbreitungseigenschaften aufweist, welche modifizierbar sind, indem sie einer bestimmten Strahlung ausgesetzt werden, wobei im Schritt (c) des Berechnens der Linse, die Linse durch Bestimmen der lokalen mechanischen Schallwellenausbreitungseigenschaften in der Linse (9) berechnet wird,
und das Verfahren einen Schritt (d) des Bildens der Linse (9) aufweist, in dessen Verlauf die Linse lokal der vorbestimmten Strahlung ausgesetzt wird, um die lokalen mechanischen Schallwellenausbreitungseigenschaften zu erhalten, die im Schritt (c) des Berechnens der Linse zu erhalten.

**18.** Verfahren zur Beschallung eines Milieus (3) mit mindestens einer aberrierenden Barriere (8) und einem im Wesentlichen homogenen inneren Teil (7), der durch die aberrierende Barriere (8) verdeckt ist,

wobei das Beschallungsverfahren die Schritte eines Verfahrens zur Herstellung einer Ultraschalllinse (9) nach einem der Ansprüche 2-17 aufweist;
wobei das Beschallungsverfahren ferner einen Emissionsschritt aufweist, in dessen Verlauf die vorbestimmte Ultraschallwelle (13a) mittels an der vorbestimmten Position platzierten Ultraschallsonde durch die Ultraschalllinse (9) emittiert wird, wobei die vorbestimmte Welle das vorbestimmte Ziel-Ultraschallwellenfeld in mindestens einer zu dem inneren Teil (7) gehörenden vorbestimmten Zone (F) erzeugt.

**19.** Verfahren zur Beschallung nach Anspruch 18, bei welchem das vorbestimmte Ultraschallwellenfeld in der mindestens einen vorbestimmten Zone (F) fokussiert wird.

**20.** Vorrichtung zur Berechnung einer Ultraschalllinse (9)

wobei die Ultraschalllinse zur Beschallung eines Milieus geeignet ist, das mindestens eine aberrierende Barriere (8) und einen im Wesentlichen homogenen inneren Teil (7) aufweist, der von der aberrierenden Barriere (8) verdeckt ist,
wobei die Berechnungsvorrichtung Einrichtungen zum Berechnen der Linse (c) aufweist, welche dazu ausgebildet sind, die Ultraschalllinse (9) unter Verwendung eines Modells des Milieus zu berechnen, welches eine anhand eine realen Abbildung des Milieus (3) erhaltene Kartierung der Ausbreitungseigenschaften der Ultraschallwellen aufweist,

wobei die Ultraschalllinse (9) dazu ausgebildet ist, wenn die Ultraschalllinse (9) zwischen einer Ultraschallsonde (2) und der aberrierende Barriere (8) angeordnet ist und wenn die Ultraschallsonde (2), welche in Bezug auf das Milieu (3) an einer vorbestimmten Position dem inneren Teil (7) in Bezug auf die aberrierende Barriere (8) gegenüberliegend platziert ist, eine vorbestimmte Ultraschallwelle (13a) emittiert, Aberrationen zu erzeugen, so dass die vorbestimmte Welle ein vorbestimmtes Ziel-Ultraschallwellenfeld in mindestens einer vorbestimmten Zone (F) erzeugt, welche zu dem inneren Teil (7) gehört.

21. Vorrichtung zur Herstellung einer Ultraschalllinse, mit einer Vorrichtung zur Berechnung einer Ultraschalllinse nach Anspruch 20 und Einrichtungen zur Bildung der Ultraschalllinse (9).

**Claims**

1. A calculation method, implemented by computer, of a suitable ultrasonic lens for insonification of a medium comprising at least one aberrating barrier (8) and a substantially homogeneous internal part (7) masked by said aberrating barrier (8),

   the calculation method comprising at least a lens calculation step (c) during which an ultrasonic lens (9) is calculated, using a model of the medium comprising a mapping of ultrasonic wave propagation properties obtained from actual imaging of the medium (3),
   the ultrasonic lens (9) being configured to, when the ultrasonic lens (9) is interposed between an ultrasound probe (2) and the aberrating barrier (8) and when the ultrasound probe (2) placed in a predetermined position relative to said medium (3) on the other side of said internal part (7) relative to said aberrating barrier (8) sends a predetermined ultrasonic wave (13a), generate aberrations such as said predetermined wave generates a predetermined objective ultrasonic wave field in at least one predetermined area (F) belonging to said internal part (7).

2. A manufacturing method for producing the lens calculated according to the method of claim 1, further comprising a lens (9) implementation step (d) calculated in the lens calculation step (c)

3. The manufacturing method according to claim 2, wherein the lens (9) implementation step (d) is done by a method chosen among three-dimensional printing of the lens (9) and digitally controlled machining of at least one block of material for forming the lens (9).

4. The manufacturing method according claim 2, wherein the lens (9) implementation step (d) comprises the following substeps:

   (d1) a substep of implementation of at least one mold done by a method chosen among three-dimensional printing of said at least one mold and digitally controlled machining of at least one block of material for forming said at least one mold;
   (d2) a substep of molding in which said lens (9) or at least one component of said lens (9) is molded in said at least one mold.

5. The method according to any one of claims 1 to 4, wherein the lens calculation step (c) is preceded by an imaging step (a) during which said mapping is done.

6. The method according to claim 5, wherein the imaging step (a) is done by x-ray or RMI imaging.

7. The method according to claim 6, wherein said cartography is done from bone density measurements.

8. The method according to any one of claims 1 to 7, wherein the lens calculation step (c) is preceded by a simulation step (b) during which:

   (b1) propagation in the medium of the predetermined ultrasonic wave (13a) from the ultrasound probe (2) disposed at said predetermined position to a chosen position of the lens (9) is calculated, and the first arrival

times $t_0(x,y)$ of said predetermined ultrasonic wave to said chosen lens (9) position are determined;

(b2) back-propagation in the medium (3) of said predetermined objective ultrasonic wave field from the predetermined area (F) to said chosen lens (9) position is simulated;

(b3) second arrival times $t_1(x,y)$ of said back-propagation of said predetermined objective ultrasonic wave field to said chosen lens (9) position are determined;

(b4) a delay law $\Delta t(x,y)$ at said chosen lens (9) position equal to the sum of the first arrival times $t_0(x,y)$ and second arrival times $t_1(x,y)$ is determined;

and during the lens calculation step (c), the ultrasonic lens (9) is calculated using said delay law $\Delta t(x,y)$, so that when the ultrasound probe (2) sends said predetermined ultrasonic wave (13a) through said ultrasonic lens, said predetermined wave reproduces the objective wave field in said area F after passing through said aberrating barrier (8).

9. The method according to claim 8, wherein the first arrival times $t_0(x,y)$ and the second arrival times $t_1(x,y)$ are determined at different control points M(x,y) belonging to a predetermined control surface (14) located at said chosen lens (9) location.

10. The method according to claim 9, wherein the control surface (14) is located between the ultrasound probe (2) and the aberrating barrier (8).

11. The method according to any one of claims 9 and 10, wherein at the lens (9) calculation step (c), an ultrasonic lens thickness e(x,y) is calculated at each point L(x,y) of the lens corresponding to a control point M(x,y) with the following formula:

$$e(x,y)= \Delta t(x,y)/(1/c-1/c_1)+ e_0$$

where:

- c is the speed of the ultrasonic wave in the medium (3) outside of the aberrating barrier (8);
- $c_1$ is the speed of the ultrasonic wave in the lens;
- $e_0$ is a real number such that e(x,y) has a has a value at each point of the lens that is positive and greater than a predetermined thickness.

12. The method according to any one of claims 9 and 10, wherein the ultrasonic wave is monochromatic and at the lens (9) calculation step (c), a lens thickness e(x,y) is calculated at each point L(x,y) of the lens corresponding to a control point M(x,y) with the following formula:

$$e(x,y)= \Delta t(x,y)/(1/c-1/c_1)+ e_0, \text{ modulo } T/(1/c-1/c_1).$$

where:

- c is the speed of the ultrasonic wave in the medium outside of the aberrating barrier;
- $c_1$ is the speed of the ultrasonic wave in the lens;
- $e_0$ is a real number such that e(x,y) has a has a value at each point of the lens that is positive and greater than a predetermined thickness;
- T is the period of the ultrasonic wave.

13. The method according to any one of claims 8 to 12, wherein at substep (b3), the arrival times $t_0(x,y)$ are determined by simulating propagation of said predetermined ultrasonic wave in a simplified model allowing an analytic calculation.

14. The method according to any one of claims 1 to 13, wherein the lens calculation step (c) considers travel times of said predetermined ultrasonic wave in the ultrasonic lens (9) and angles of incidence of said predetermined ultrasonic wave at the surface of the ultrasonic lens (9).

15. The method according to any one of claims 1 to 14, wherein the lens calculation step (c) considers the entire propagation of said predetermined ultrasonic wave, including echoes between the ultrasound probe (2), the ultrasonic lens (9) and the aberrating barrier (8).

**16.** The method according to any one of claims 1 to 15, wherein the lens calculation step (c) is done iteratively in order to optimize the ultrasonic energy transmission yield in the predetermined area (F), using an analytic or numerical model.

**17.** The method according to any one of claims 1 to 16, wherein the lens (9) is made of a material having acoustic wave propagation properties modifiable by exposure to a predetermined radiation,

during lens calculation step (c), the lens is calculated by determining local mechanical characteristics of acoustic wave propagation in said lens (9),
and the method comprises a lens (9) implementation step (d) during which said lens is locally exposed to said predetermined radiation for obtaining the local mechanical characteristics of acoustic wave propagation determined in lens calculation step (c).

**18.** An insonification method of a medium (3) comprising at least one aberrating barrier (8) and a substantially homogeneous internal part (7) masked by said aberrating barrier (8),

the insonification method comprising the steps of a manufacturing method of an ultrasonic lens (9) according to any of claims 2 to 17;
the insonification method further comprising a step of emission during which said predetermined ultrasonic wave (13a) is sent through the ultrasonic lens (9) by an ultrasonic wave placed in said predetermined position said predetermined wave generating said predetermined objective ultrasonic wave field in said predetermined area (F) belonging to said internal part (7).

**19.** The insonification method according to claim 18, wherein said predetermined ultrasonic wave field is focused in said internal part in at least one predetermined area (F).

**20.** A calculation device of an ultrasonic lens (9),

the ultrasonic lens being suitable for insonification of a medium comprising at least one aberrating barrier (8) and a substantially homogeneous internal part (7) masked by said aberrating barrier (8),
the calculation device comprising lens calculation medium (c) configured to calculate said ultrasonic lens (9), using a model of the medium comprising a mapping of ultrasonic wave propagation properties obtained from actual imaging of the medium (3),
said ultrasonic lens (9) being configured to, when it is interposed between an ultrasound probe (2) and the aberrating barrier (8) and when the ultrasound probe (2) placed in a predetermined position relative to said medium (3) on the other side of said internal part (7) relative to said aberrating barrier (8) sends a predetermined ultrasonic wave (13a), generate aberrations such as said predetermined wave generates a predetermined objective ultrasonic wave field in at least one predetermined area (F) belonging to said internal part (7).

**21.** Device for manufacturing an ultrasonic lens, comprising a calculation device of an ultrasonic lens according to claim 20 and means for producing the ultrasonic lens (9).

FIG. 1

FIG. 2

FIG. 3

FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004019784 A1

**Littérature non-brevet citée dans la description**

- **Y. KIM et al.** *Rapid prototyping fabrication of focused ultrasound transducers,* 2014